# EUROPEAN PATENT SPECIFICATION

(11) **EP 4 422 513 B1**
(45) Date of publication and mention of the grant of the patent: **20.05.2026**
(21) Application number: 22812487.1
(22) Date of filing: 25.10.2022
(51) Int. Cl.: A61B 17/00

(54) **LARGE BORE VASCULAR CLOSURE SYSTEM**
GEFÄSSVERSCHLUSSSYSTEM MIT GROSSEM DURCHMESSER
SYSTÈME DE FERMETURE VASCULAIRE DE GRAND CALIBRE

(30) Priority: 25.10.2021 EP 21204538
(43) Date of publication of application: 04.09.2024
(73) Proprietor: SpioRAD Medical Limited, Cloghran Co. Dublin K67 A9P8 (IE)
(72) Inventor: O'MALLEY, Judi, Portmarnock, Co. Dublin D13 Y5Y0 (IE); ARNOUS, Samer, Crecora, Co. Limerick, V94 E6X4 (IE); NOLAN, Roisin, Galway, H91 ND8N (IE); STRYCHARSKI, Lukasz, Doughiska, Galway H91E2WA (IE); WUJANICZ, Stefan, Craughwell, Co. Galway H91DY6T (IE)
(74) Representative: Purdylucey Intellectual Property
(86) International application number: PCT/EP2022/079846
(87) International publication number: WO 2023/072972

(56) References cited:
- EP-A1- 3 777 716
- WO-A1-00/78226
- US-A1- 2007 118 176
- US-A1- 2012 016 412
- US-A1- 2012 316 602
- US-B2- 6 949 116

## Description

### Field of the Invention

The present invention is defined in the appended claims and relates to a large bore vascular closure system.

### Background to the Invention

In percutaneous medical procedures, an opening may be created in a wall of a blood vessel to allow for the insertion of various medical devices which can be navigated through the blood vessel to a site to be treated. For example, after initial access into the blood vessel is obtained, a medical device may be inserted through the tissue tract created between the skin, or epidermis, of the patient down through the subcutaneous tissue and into the opening formed in the blood vessel. The medical device may then be navigated through the blood vessel to the treatment site.

Once the procedure is completed, the medical device(s) or other equipment introduced into the blood vessel may be retracted from the body through the blood vessel, out the opening in the wall of the blood vessel, and out through the tissue tract. The physician or other medical technician is presented with the challenge of trying to close the opening in the blood vessel and/or the tissue tract formed in the epidermis and subcutaneous tissue. Several different device structures, assemblies, and methods are known for closing the opening in the blood vessel and/or tissue tract, each having certain advantages and disadvantages. There is an ongoing need to provide alternative medical devices as well as alternative methods for manufacturing and/or using medical devices. US2007118176A1 discloses an occluder for a biological defect, such as an atrial septal defect (ASD) or a patent foramen ovale (PFO). The occluder is at least partially formed of a radiopaque, bioabsorbable material. In some embodiments, the occluder is formed from a tube, which is cut to produce struts in each side. Upon the application of force, the struts deform into loops. The radiopaque, bioabsorbable material is a blend of a biocompatible radiopaque material with a bioabsorbable material. EP3777716A1 provides an occlusion device and a preparation method therefor. The occlusion device includes a framework and blood-blocking membranes. The framework includes a waist and barbs connected to at least one end of the waist. The blood-blocking membranes are secured at least one end of the waist and/or at inner sides of the barbs. The framework and the blood-blocking membranes together define a cavity, and an injection orifice is formed in the framework or the blood-blocking membranes. This occlusion device employs a structure allowing it to have a smaller size and thus be able to be loaded in a thinner delivery sheath having a smaller diameter. Additionally, the amount of material used is reduced, lowering the incidence of post-implantation complications and resulting in cost savings. Taking advantage of the behavior of a degradable hydrogel, defects with irregular shapes, such as those with relatively narrow edge rims and complicated shapes, can be filled up, thus extending the scope of application of interventional treatment to cardiac septal defects. US2012316602A1 discloses a septal occluder, such as one made from a polymer tube, which can have portions on either side of a patent foramen ovale (PFO) or other septal defect. The portions on either side can be held in place with a catching mechanism that can take one of many forms. The tube can be made of bioresorbable materials. A septal occluder, such as one made from a polymer tube, can have portions on either side of a patent foramen ovale (PFO) or other septal defect. The portions on either side can be held in place with a catching mechanism that can take one of many forms. The tube can be made of bioresorbable materials. WO0078226A1 discloses a tool is provided for inserting and mating two plug members of a sealing device for closing a wound in the wall of a vessel by placing the distal plug member of the sealing device inside the vessel and the proximal plug member on the outside of the vessel. The distal plug member is provided with an elongated retracting means extending from the distal plug member. The tool comprises a gear mechanism coupled to the retracting means for converting a movement of the tool in a proximal direction away from the wound, when the distal plug member is anchored in the vessel, to a pushing movement for moving the proximal plug member in a direction towards the distal plug member in response to a stretching force in the retracting means.

Current closure devices on the market do not extend to be inclusive of full-size requirement. Also, complication rate is high major & minor (12% - 29%) with problematic usability known, often resulting in numerous devices being used per procedure. Issues of haemostasis results in blood loss, and risks of embolization from intravascular devices result in acute limb ischemia risk limb loss often resulting in immediate surgical escalation and intervention.

It is an object of the invention to overcome at least one of the above-referenced problems.

### Summary of the Invention

The Applicant has addressed the problem of the prior art by providing a large bore closure system that is fully extravascular, obviating the risks of embolization. This is achieved by providing an occlusion device configured for deployment in a tissue tract proximal of an opening in a blood vessel such that upon deployment no part of the device is disposed in the blood vessel. The occlusion device is radially adjustable from an elongated delivery configuration to a deployed squat and radially expanded configuration where the device anchors in a tissue tract. Typically, a bioresorbable injectable filler material is provided for injection into the lumen of the occlusion device to occlude the through lumen. The filler material helps the occlusion device maintain is deployed configuration and also seeps through the sidewall of the occlusion device to adhere to the wall of the tissue tract to help anchor the deployed occlusion device in position. The device generally comprises a waist section that is configured to expand radially outwardly when the device is compressed axially. Due to the radial expansion feature, the device is adaptable for use with tissue tracts of varying bore size, for example from 12F to 26F (4.0 mm to 8.7 mm) Thus, the large bore vascular closure system of the invention provides decreased risk by not having anything within the artery to cause embolization, furthermore it can be employed to close tissue tracts generated by the largest percutaneous access devices on the market. In one embodiment, the system comprises an introducer device configured to deliver the occlusion device into the tissue tract, and an occlusion device deployment system comprising a control wire (e.g., a suture) configured to deploy the occlusion device in the tissue tract. The introducer device generally comprises a through lumen for receipt of a guidewire and to allow delivery of the injectable filler material into the lumen of the deployed occlusion device. The control wire may be coupled to the occlusion device such that retraction of the control wire effects deployment of the occlusion device. The deployment system may comprise an actuator coupled to the introducer device and configured upon actuation to retract the control. The actuator may be a rotor. The actuator may be coupled to a handle of the introducer device. The control wire may be attached to a distal end of the device whereby retraction of the control wire pulls the distal end of the device closer to the proximal end of the device to deploy the device. The control wire may also extend into the device through an aperture in the proximal end of the device, loop through apertures in the distal end, and extend out of the device through a second aperture in the proximal end of the device. The control wire generally includes at least one anchor element (e.g., a bead) and at least one friction fit brake element (e.g., a bead) that are provided on the control wire to enable the device to be deployed into the squat radially expanded configuration upon retraction of the control wire and maintained in the deployed configuration.

The present invention is solely defined in the appended claims.

In a first aspect, the disclosure provides a large bore extra-vascular closure system comprising:
an introducer device having a through lumen and a handle optionally comprising an actuator;
an occlusion device having a distal end, a proximal end, a sidewall section (typically comprising a waist section configured for outward inflection upon axial compression of the device), and a through lumen, wherein the occlusion device is configured for adjustment from an elongated delivery configuration in which the occlusion device is dimensioned to fit within the lumen of the introducer device and a squat deployed configuration in which the waist section of the occlusion device is expanded radially outwardly.

In any embodiment, the through lumen of the introducer device has a diameter of any least 0.5 mm, for example 0.5 to 2.0 mm or 0.5 to 1.5 mm.

In any embodiment, the control wire is a suture, for example a bioresorbable suture.

In any embodiment, the sidewall section of the bioresorbable occlusion device has an open framework. This allows the injectable filler material to seep out of the occlusion device and adhere to the walls of the tissue tract to help anchor the occlusion device in position.

In any embodiment, the open framework of the bioresorbable occlusion device comprises a plurality of struts.

In any embodiment, the plurality of struts are arranged around a longitudinal axis of the bioresorbable occlusion device.

In any embodiment, the plurality of struts extend between the proximal and distal ends of the device.

In any embodiment, the system comprises a control wire operably connecting the actuator of the introducer device and the distal end of the occlusion device, wherein the actuator is configured upon actuation to pull the control wire proximally to deploy the occlusion device.

In any embodiment, the occlusion device and/or the control wire are bioresorbable.

In any embodiment, the system comprises a plurality of control wires connecting the actuator of the introducer device and the distal end of the occlusion device.

In any embodiment the control wires are connected to the occlusion device in a symmetrical manner (e.g., connected to opposite sides) to balance the deployment of the device.

In any embodiment, the control wire extends through a proximal aperture in the proximal end of the occlusion device and through a distal aperture in the distal end of the occlusion device.

In any embodiment, the proximal aperture is smaller than the distal aperture.

In any embodiment, the control wire comprises an anchor element (e.g., a first bead) that cannot pass through the distal or proximal aperture. The anchor may be positioned on the control wire outside of the occlusion device and bear against an end of the occlusion device when the control wire is pulled proximally. The anchor element may be a bead or knot or other suitable formation on the control wire. The anchor element is generally sufficiently large relative to the aperture to prevent it passing through the aperture.

In any embodiment, the control wire comprises a brake element (e.g., a second bead) dimensioned for a friction fit through the distal or proximal aperture. The brake element is dimensioned for a friction fit through an aperture in the distal or proximal end of the device, generally the proximal end. The size of the brake element and aperture are chosen such that the brake can be forced through the aperture by pulling the control wire, but that when it is released, the tension on the deployed device is not sufficient to pull the brake element back through the aperture. Thus, the control wire can be pulled to deploy and tension the occlusion device, and the brake element will maintain the occlusion device in the deployed configuration unless and until it is released. The control wire may include a plurality of brake elements, for example 2 to 6 or 3 to 5.

In any embodiment, the system comprises a plurality of second bead elements disposed on the control wire intermediate the ends of the occlusion device when the occlusion device is in the elongated delivery configuration.

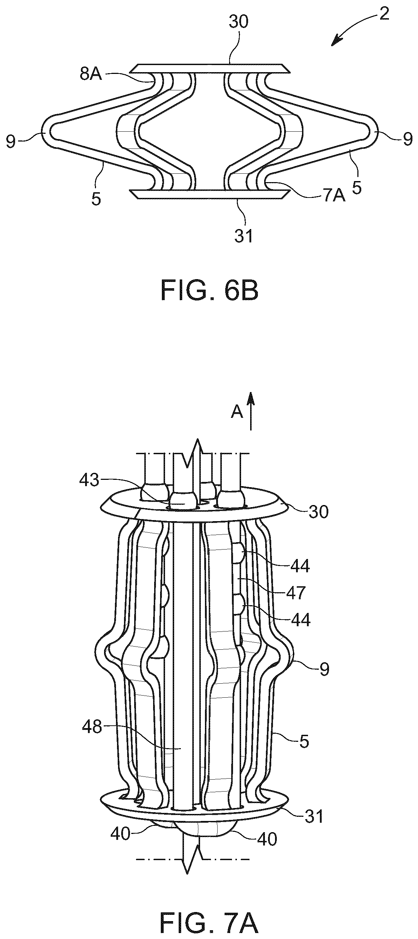

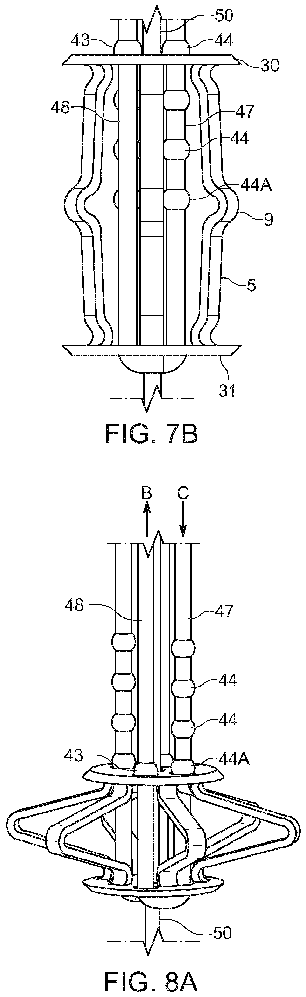

In one embodiment, the first bead element is disposed on the control wire distally of the distal aperture and is dimensioned such that it cannot pass through the distal aperture. This embodiment is illustrated in Figures 7A and 7B.

In another embodiment, the distal end of the occlusion device comprises two distal apertures and the proximal end of the device comprises two proximal apertures, in which the control wire extends through a first of the proximal apertures, loops through the distal apertures in the distal end of the device and extends through a second of the proximal apertures.

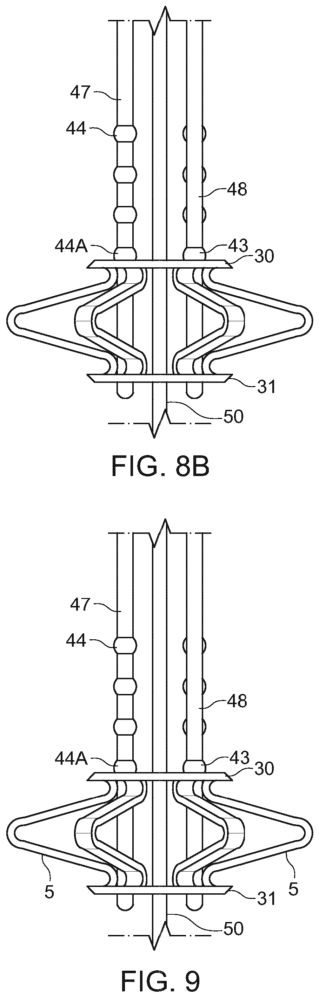

In any embodiment, the first bead element is disposed on the control wire proximally of the proximal aperture and is dimensioned such that it cannot pass through the distal aperture. This embodiment is illustrated in Figures 8 to 11.

In any embodiment, the actuator is a rotor and the control wire is mounted to the rotor whereby rotation of the rotor effects movement of the control wire relative to the introducer device.

In any embodiment, the control wire is a closed loop.

In any embodiment, the control wire has free ends coupled to the actuator.

In any embodiment, the system is configured such that rotation of the rotor in a first direction causes the occlusion device to deploy into the squat radially expanded configuration and rotation of the rotor in a second direction causes the occlusion device to return to the elongated delivery configuration.

In any embodiment, the rotor comprises a graduated scale disposed on a circumference of the rotor indicating a range of large bore sizes.

In any embodiment, the sidewall section of the occlusion device is defined by a plurality of elongated struts arranged around a longitudinal axis of the occlusion device. Generally, the system comprises a flexible fluid impermeable sheath configured to embrace the occlusion device. Preferably, the sheath is bioresorbable.

In any embodiment, the waisted section of the sidewall is formed by an outward inflection zone disposed intermediate the ends of each elongated strut configured to allow the strut fold radially outwardly during deployment.

In any embodiment, each of the elongated struts comprises an inward inflection zone disposed at each end of the strut configured to allow an adjacent section of the strut bend radially outwardly during deployment.

In any embodiment, the system comprises an injectable filler material configured for injection into the hollow lumen of the occlusion device when deployed. The invention also relates to a system of the invention comprising the injectable filler material disposed in the hollow lumen of the occlusion device when deployed. The filler is generally a bioresorbable material. The filler may be a thermosetting material that is sufficiently fluid at room temperature to be injected into the occlusion device and that hardens at body temperature to form a fluid impermeable tamponade disposed in the occlusion device. Examples of filler materials include hydrogels and collagen materials. The injectable filler material may be a tissue adhesive such as a cyanoacrylate glue (Covidien) or a collagen-based tissue adhesive.

In any embodiment, the system comprises a syringe device comprising a delivery conduit configured to extend through the through lumen of the introducer device to deliver injectable filler material into the through lumen of the bioresorbable occlusion device when the bioresorbable occlusion device is deployed.

In any embodiment, the delivery conduit of the syringe device comprises a non-stick coating configured to inhibit the injectable filler material sticking to an inside of the delivery conduit. In any embodiment, the non-stick coating comprises or consists essentially of glucose.

In any embodiment, the system comprises a balloon disposed inside a hollow lumen of the frame configured to receive and contain the injectable filler material.

In any embodiment, the system comprises a guidewire in which the introducer device is configured to be mounted on the guidewire.

In any embodiment, the occlusion device is formed from a synthetic homopolymer or a co-polymer formed of synthetic homopolymers.

In any embodiment, the introducer device comprises an occlusion device ejection module actuatable to expose the occlusion device proud of a distal end of the introducer device.

In any embodiment, the occlusion device has a length of 4.0 to 12.0 mm in a delivery configuration and
In any embodiment, the occlusion device has a length of 0.5 to 4.0 mm in a deployed configuration.

In any embodiment, the occlusion device has a maximum diameter of 4.0 to 8.7 mm in a delivery configuration
In any embodiment, the occlusion device has a maximum diameter of 8.0 to 8.7 mm in a deployed configuration.

In any embodiment, the occlusion device is configured to radially expand from a maximum diameter in a delivery configuration D1 to a maximum diameter in a deployed configuration D2, in which D2 is at least 25%, 50%, 75%, 100%, 150% or 200% greater than D1.

In any embodiment, D2 is at least 25% - 200%, 25% - 150%, 50% to 150%, or 50% to 100% greater than D1.

In any embodiment, the occlusion device includes a locking module configured to lock the occlusion device in a deployed configuration. The locking module may comprise a locking member attached to one end of the occlusion device and having a free end configured to engage an opposite end of the device when the device is deployed. The locking member may be an arm. The free end of the locking member may include a hook element and the opposite end of the device may include a hook receiving element configured to receive the hook element when the device is deployed. The locking member may be attached to a distal end of the occlusion device.

In any embodiment, the flexible fluid impermeable sheath is configured to fully enclose the occlusion device.

In any embodiment, the occlusion device comprises a shape-memory material such as nitinol. In any embodiment, the occlusion device is self-expansible and biased into a deployed configuration.

In another aspect, the invention provides an occlusion device to close a large bore tissue tract in a subject comprising a body configured for adjustment from a delivery configuration in which the occlusion device is dimensioned to fit within the lumen of the introducer device and a squat deployed configuration in which the occlusion device is expanded radially outwardly.

In any embodiment, the fluid impermeable occlusion device is configured for adjustment between an elongated delivery configuration and a squat radially expanded deployed configuration.

In any embodiment, the body of the fluid impermeable occlusion device has a distal end, a proximal end, a sidewall section comprising a waist section configured for outward inflection upon axial compression of the device, and a through lumen.

In any embodiment, the occlusion device is bioresorbable.

In any embodiment, the occlusion device comprises a flexible fluid impermeable sheath enclosing the body. In any embodiment, the occlusion device is bioresorbable.

In any embodiment, the sidewall section of the body has an open framework. This allows the injectable filler material seep through the sidewall section and adhere to the wall of the tissue tract.

In any embodiment, the sidewall section of the body comprises a plurality of elongated struts, typically arranged around a longitudinal axis of the occlusion device.

In any embodiment, the occlusion device has 4-10, 4-8, 4-6, 6-10 or 6-8 struts.

In any embodiment, the waisted section of the sidewall is formed by an outward inflection zone disposed intermediate the ends of each elongated strut configured to allow the strut fold radially outwardly during deployment.

In any embodiment, at least one and generally all the elongated struts comprise an inward inflection disposed at one or both ends of the strut configured to allow an adjacent section of the strut bend outwardly during deployment.

In any embodiment, the occlusion device comprises an injectable filler material disposed in the hollow lumen of the occlusion device. The filler is generally a commercially available bioresorbable material. The filler may be a thermosetting material that is sufficiently fluid at room temperature to be injected into the occlusion device and that hardens at body temperature to form a fluid impermeable tamponade disposed in the occlusion device. Examples of filler materials include hydrogels and collagen materials.

In any embodiment, the occlusion device comprises a balloon disposed inside a hollow lumen of the frame configured to receive and contain the injectable filler material.

In any embodiment, the occlusion device comprises or is formed from a synthetic homopolymer or a co-polymer formed of synthetic homopolymers.

In any embodiment, the occlusion device has a length of 0.4-8.0 mm in a delivery configuration and a length of 4.0-12.0 mm in a deployed configuration.

In any embodiment, the occlusion device has a maximum diameter of 0.4-9.3 mm in a delivery configuration and a maximum diameter of 8.0-10.7 mm in a deployed configuration.

In any embodiment, the occlusion device comprises a locking module configured to lock the occlusion device in a deployed configuration. The locking module may comprise a locking member attached to one end of the occlusion device and having a free end configured to engage an opposite end of the device when the device is deployed. The locking member may be an arm. The free end of the locking member may include a hook element and the opposite end of the device may include a hook receiving element configured to receive the hook element when the device is deployed. The locking member may be attached to a distal end of the occlusion device.

In any embodiment, the flexible fluid impermeable sheath is configured to fully enclose the occlusion device.

In any embodiment, the occlusion device comprises a shape-memory material such as nitinol. In any embodiment, the frame comprises or is formed from nitinol. In any embodiment, the occlusion device is self-expansible and biased into a deployed configuration.

In another aspect, the disclosure provides a method not forming part of the present invention of closing a large bore tissue tract in a subject comprising:
providing a system according to the disclosure;
advancing a distal end of the introducer device into the large bore tissue tract in the subject over a guidewire to a position proximal of a blood vessel;
actuating the introducer device to advance the occlusion device into the large bore tissue tract proximally of the artery or vein; and
actuating the actuator on the handle of the introducer device to pull the control wire proximally to deploy the occlusion device.

In any embodiment, the method is for closing a tissue tract in fluid communication with an opening in a blood vessel in a subject.

In any embodiment, the method is for closing a tissue tract in fluid communication with an opening in a femoral artery in a subject.

In any embodiment, the occlusion device is deployed 0.5-2.0 cm distally of the opening in the blood vessel.

In any embodiment, the position of the occlusion device is monitored using imaging, typically contrast imaging.

In any embodiment, the occlusion device is deployed extra-vascularly, typically just proximally of the adventitial layer of an artery or vein.

In any embodiment, the occlusion device is radially expanded to a diameter that is greater than a diameter of the tissue tract, typically at least 5%, 10%, 15% or 20% greater.

In any embodiment, occlusion of the tissue track is monitored using imaging, typically contrast imaging.

In any embodiment, the method includes a step of injecting an injectable filler material into the occlusion device after the occlusion device has been deployed.

In any embodiment, the filler material is a semi-solid material and/or a thermosetting material.

In any embodiment, the filler material comprises tissue adhesive properties when injected.

In any embodiment, the introducer device is advanced into the tissue tract over a guidewire.

In any embodiment, the control wire has an anchor element disposed distally of the distal end of the occlusion device, wherein the step of pulling the control wire distally causes the distal end of the occlusion device to be pulled proximally towards the proximal end of the device to deploy the occlusion device.

In any embodiment, the occlusion device comprises two distal apertures and the proximal end of the device comprises two proximal apertures, in which the control wire extends through a first of the proximal apertures, loops through the distal apertures in the distal end of the device and extends through a second of the proximal apertures, and wherein the control wire has an anchor element disposed proximally of the proximal end of the occlusion device, wherein the step of pulling the control wire distally causes the distal end of the occlusion device to be pulled proximally towards the proximal end of the device to deploy the occlusion device.

In any embodiment, the control wire comprises a brake element disposed in between the distal end and proximal end of the occlusion device, wherein the step of pulling the control wire proximally causes the brake element to squeeze through the proximal aperture in the proximal end of the device and resist the tensioned occlusion device returning to the elongated delivery configuration.

In any embodiment, the actuator is a rotor to which the control wire is coupled such that rotation of the rotor in a first direction causes the occlusion device to deploy into the squat radially expanded configuration and rotation of the rotor in a second direction causes the occlusion device to return to the elongated delivery configuration.

In any embodiment, the guidewire is removed leaving the occlusion device in-situ in the tissue track.

In any embodiment, the introducer device comprises a distal end to receive the occlusion device and an ejection module to expose the occlusion device proud of the distal end of the introducer device.

In any embodiment, the introducer device comprise a distal conduit (comprising the through lumen)and an outer retractable sheath configured for axial adjustment relative to the distal conduit from a delivery configuration in which a distal end of the outer retractable sheath extends distally of the distal end of the distal conduit (e.g. providing a space for receipt of the occlusion device) and a deployment configuration where the outer retractable sheath is retracted distally (e.g. to expose the occlusion device to the surrounding wall of the tissue tract.).

In any embodiment, the introducer device comprises one or more control wire lumens to house the or each control wires.

In any embodiment, the introducer device comprises a window (reveal) to allow access to the or each control wire lumen allowing the control wire(s) to be cut after the occlusion device has been deployed and anchored.

In any embodiment, the windows are provided on a proximal section of the introducer device that can be accessed by a user during use.

In any embodiment, the distal conduit part of the introducer device has a central through lumen (for receipt of the guidewire and the injectable filler material delivery conduit) and two (typically crescent-shaped) control wire lumen, each of which receives an ascending and descending parts of a control wires.

In any embodiment, the introducer device comprises a window (reveal) on each side, each window providing a user access to the control wires to allow them to be cut when the occlusion device is deployed and anchored.

In any embodiment, the distal conduit part of the introducer device has a central through lumen (for receipt of the guidewire and the injectable filler material delivery conduit) and four control wire lumens, each of which receives an ascending or descending parts of each control wire.

In any embodiment, the introducer device comprises a control wire release mechanism configured upon actuation to uncouple the control wires from the introducer device (e.g., from the actuator of the introducer device) to allow the occlusion device and introducer device to be uncoupled and the introducer device to be retracted and removed.

In any embodiment, the distal conduit of the introducer device has a single lumen configured to receive the guidewire, filler material conduit, and the control wires.

In any embodiment, the distal conduit comprises a cylindrical body, and end cap, and a coupling element to couple the cylindrical body and end cap configured to allow axial movement of the cylindrical body relative to the end cap between a delivery position in which the end cap is attached to a distal end of the cylindrical body and an uncoupling position in which the end cap is axially spaced from an end of the cylindrical body.

In any embodiment, the coupling element is a central strut.

In any embodiment, the end cap is dimensioned to receive a distal end of the occlusion body in a nesting relationship.

In any embodiment, the end cap comprises a central aperture (or a plurality of apertures) to receive the control wires, guidewire and injectable filler material delivery conduit.

In any embodiment, the introducer device typically comprises an actuator to axially adjust the position of the cylindrical body relative to the end cap to expose the control wires to allow them to be cut.

Other aspects and preferred embodiments of the invention are defined and described in the other claims set out below.

### Brief Description of the Figures

**FIG. 1** is a side elevational view of an occlusion device forming part of a large bore extra-vascular closure system of the invention, shown in an elongated delivery configuration.
**FIG.2** is a side elevational view of an occlusion device forming part of a large bore extra-vascular closure system of the invention, shown in a squat deployed configuration.
**FIG. 3A** is a perspective view of a frame forming part of the occlusion device of Figure 1, shown in an elongated delivery configuration.
**FIG. 3B** is a perspective view of a flexible fluid impermeable sheath forming part of the occlusion device of Figure 1, shown in an elongated delivery configuration.
**FIG. 4A** is a perspective view of a frame forming part of the occlusion device of Figure 1, shown in a squat deployed configuration.
**FIG. 4B** is a perspective view of a flexible fluid impermeable sheath forming part of the occlusion device of Figure 1, shown in a squat deployed configuration.
**FIG. 4C** is a perspective view of the occlusion device of Figure 1, shown in a squat deployed configuration.
**FIG. 5A** is a perspective view of an occlusion device according to the invention in an elongated delivery configuration.
**FIG. 5B** is a side elevational view of the occlusion device of Figure 5A.
**FIG. 5C** is a top plan view of the occlusion device of Figure 5A.
**FIG. 6A** is a perspective view of the occlusion device of Figure 5A in a squat deployed configuration.
**FIG. 6B** is a side elevational view of the occlusion device of Figure 5A in a squat deployed configuration.
**FIG. 7A** is a perspective view of part of a system of the invention comprising the occlusion device of Figure 5A and two control wires each extending down through the proximal end, looping through the distal end and extending up through the proximal end and having anchor beads and friction fit beads.
**FIG. 7B** is a side elevational view of the system of Figure 7A.
**FIG. 8A** is a perspective view of the system of Figure 7A after the control wire has been retracted pulling the part of the control wire with the friction fit beads upwardly to compress the occlusion device into the deployed configuration. The last friction bead disposed just proximally of the aperture in the proximal end plate serves to maintain the device in the tensioned squat configuration.
**FIG. 8B** is a side elevational view of the system of Figure 8A.
**FIG. 9** illustrates how the device can be unlocked and returned to an expended delivery configuration by pulling one side of the control wire whereby the other side of the control wire with the friction fit beads is pulled downwardly releasing the tension on the occlusion device.
**FIG. 10A** is a sectional view of an occlusion device according to the invention in an elongated delivery configuration mounted on a guidewire and positioned in a tissue tract just proximal of an opening in a femoral vein.
**FIG. 10B** is a sectional view of the occlusion device of Figure 5A after deployment fluidically occluding the tissue tract.
**FIGS. 11A to 11C** are illustrations of an occlusion device according to the invention with a locking mechanism configured to lock the occlusion device in a deployed configuration.
**FIGS. 12A to 12D** are illustrations of a system according to the invention delivering an occlusion device into a tissue tract (Figs. 12A and 12B), deploying the occlusion device (Fig. 12C), and being withdrawn to leave the occlusion device in-situ (Fig. 12D).
**FIG. 13** is a schematic side elevational view of an occlusion device is a deployed configuration just proximal of an adventitial layer of the femoral artery.
**FIG. 14A** is a perspective view of a system of the invention including an occlusion device in an elongated delivery configuration, the distal end of an introducer device, and two control wires (sutures) coupling the occlusion device and the actuator (not shown) of the occlusion device.
**FIG. 14B** is an end elevational view of the distal conduit part of the introducer device of Figure 14A.
**FIG. 15A** is a perspective view of an alternative embodiment of a system of the invention including an occlusion device in an elongated delivery configuration, the distal end of an introducer device, and two control wires (sutures) coupling the occlusion device and the actuator (not shown) of the occlusion device.
**FIG. 15B** is an end elevational view of the distal conduit part of the introducer device of Figure 15A.
**FIG. 16** is a perspective view of an alternative embodiment of a system of the invention including an occlusion device in an elongated delivery configuration, the distal end of an introducer device, and two control wires (sutures) coupling the occlusion device and the actuator (not shown) of the occlusion device.
**FIG. 17** is a perspective view of an alternative embodiment of a system of the invention including an occlusion device in an elongated delivery configuration, the distal end of an introducer device, and two control wires (sutures) coupling the occlusion device and the actuator (not shown) of the occlusion device.

### Detailed Description of the Invention

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa*. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g., a feature, element, characteristic, property, method/process step or limitation) or group of integers (e.g., features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g., the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g., the administration of an agent to a subject) which prevents or delays the onset or progression of a disease or reduces (or eradicates) its incidence within a treated population. In this case, the term treatment is used synonymously with the term "prophylaxis".

In the context of treatment and effective amounts as defined above, the term subject (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

As used herein, the term "tissue tract" refers to a bore in tissue providing fluid communication from a body surface to an opening in a blood vessel.

As used herein, the term "large bore" refers to bore having a French size of 12F (4.0 mm) to 26F (8.7mm) .341IN (8.7mm) or greater.

As used herein, the term "extra-vascular" as applied to the closure device refers to a closure device configured to implantation in a tissue tract proximal of a blood vessel.

As used herein, the term "occlusion device" refers to a device that is fluid impermeable and is radially expansible from a delivery configuration to a radially expanded deployed configuration to occlude a lumen such as a tissue tract. The device may comprise a frame and a fluid impermeable sheath. The sheath may fully enclose the frame. The frame may be compressible from an elongated delivery configuration to a compressed squat configuration having a maximum diameter greater that a maximum diameter in the delivery configuration. The frame may have a distal end, a proximal end, and a sidewall section comprising a radially outwardly foldable waist section. The sidewall section may comprise a plurality of elongated struts connecting the distal and proximal ends. The waist may be provided by the elongated struts having an outward inflection zone disposed intermediate the ends of the strut configured to allow the strut fold radially outwardly during deployment. The elongated struts may comprise an inward inflection zone disposed at each end of the strut configured to allow an adjacent section of the strut bend radially outwardly during deployment. The frame may be formed of a bioresorbable material. The frame may be 3-D printed from an extruded material. The frame may be formed from a bioresorbable homopolymer or co-polymer of homopolymers. The distal end may comprise a peripheral flange section that extends radially outwardly of the distal end of the sidewall having a diameter greater than a diameter of the proximal end, for example 1.5, 2.0 or 2.5 times greater. The distal end may be formed from an expansible material such as a firm sponge material. This allows the peripheral flange section of the distal end of the occlusion device to be compressed to fit into the introducer device and then expand radially outwardly when the occlusion device is released from the introducer device. The wide distal end of the deployed occlusion device provides a greater surface area for abutting the adventitial layer of the blood vessel.

As used herein, the term "introducer device" refers to a device configured to deliver an occlusion device into a tissue tract and comprises a distal conduit dimensioned for advancement into the tissue tract having a through lumen configured to receive the occlusion device in a delivery configuration. The introducer device generally includes a deployment module for the occlusion device, for example a rotor which is connected to a distal end of the occlusion device by a connecting element such as a wire or suture whereby the rotor can be rotated to retract the connecting element to pull the distal end of the occlusion device proximally to deploy the occlusion device. The introducer device generally also includes an ejection module configured to expose the occlusion device proud an end of the distal conduit. The ejection module may comprise an outer retractable sheath configured for axial adjustment relative to the distal conduit from a delivery configuration in which a distal end of the outer retractable sheath extends distally of the distal end of the distal conduit (e.g. providing a space for receipt of the occlusion device) and a deployment configuration where the outer retractable sheath is retracted distally (e.g. to expose the occlusion device to the surrounding wall of the tissue tract.). The introduce device may comprise one or more control wire lumens to house the or each control wires. The introducer device may include a window (reveal) to allow access to the control wire lumen allowing the control wires to be cut after the occlusion device has been deployed and anchored. Generally, the windows are provided on a proximal section of the introducer device that can be accessed by a user during use.

As used herein, the term "control wire" refers to an elongated element that connects the actuator of the introducer device and the occlusion device. It may be provided by a suture. The control wire is generally bioresorbable. The control wire may be a closed loop that is coupled with the actuator, or a wire with free ends that are coupled to the actuator. Typically, the or each control wire is a suture.

The term "anchor element" refers to as formation on the control wire such as a bead or knot that is oversized relative to an aperture through which the control wire is threaded to prevent the passage of the anchor element through the aperture and thereby prevent the control wire being passed through the aperture in a defined direction.

The term "brake element" refers to as formation on the control wire such as a bead or knot that is dimensioned relative to an aperture through which the control wire is threaded to allow the brake element squeeze through the aperture when the wire is pulled by a user, but that prevents the brake element passing back through the aperture due to forces exerted by the tensioned device. The brake element functions to lock the occlusion device in a specific deployed configuration until such time as the user unlocks the control wire by pulling the control wire in an opposite direction to force the brake element back through the aperture in an opposite direction.

The term "injectable filler material" refers to a material that is injectable through the through lumen in the introducer device and forms a fluid impermeable tamponade (e.g., a plug) in the lumen of the occlusion device to prevent blood flow through the deployed occlusion device. Examples include collagen filler materials and cyanoacrylate glues (Covidien). The filler is generally a bioresorbable material and is adsorbed by the body over time. The filler is generally an expansible material. The filler may be a thermosetting material that is sufficiently fluid at room temperature to be injected into the occlusion device and that hardens or increases in viscosity at body temperature to form the fluid impermeable tamponade disposed in the occlusion device. Other examples of filler materials include hydrogels. Typically, the filler material has a viscosity of 30 cP to 1500 cP (0.03 Pa·s to 1.5 Pa·s) measured using a Brookfield viscometer at room temperature and ambient pressure. Typically, the filler material comprises a polymer/monomer such as collagen or cyanoacrylate and an aqueous solvent. Typically, the filler material comprises 3.0mg/ml to 9.9mg/ml polymer (or monomer).

### Exemplification

The disclosure will now be described with reference to specific Examples. These are merely exemplary and for illustrative purposes only: they are not intended to be limiting in any way to the scope of the monopoly claimed or to the invention descrived, which is defined solely in the appended claims. These examples constitute the best mode currently contemplated for practicing the disclosure.

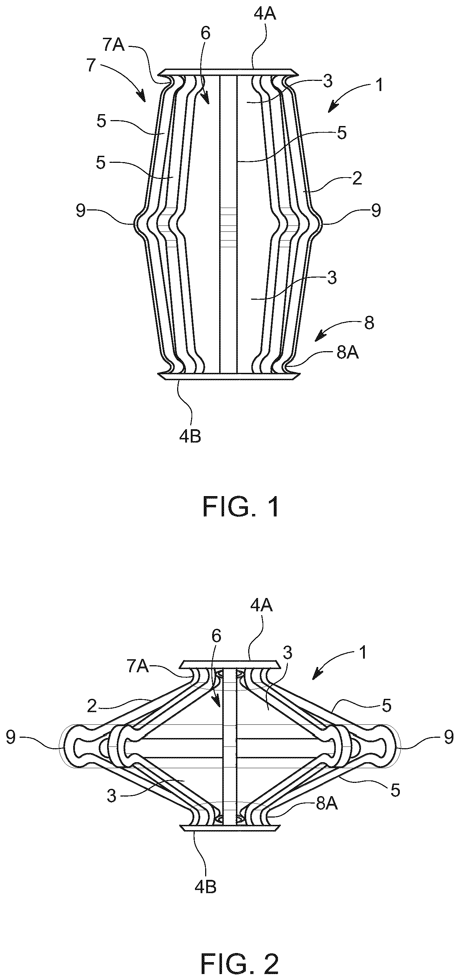

Referring to the drawings, and initially to Figures 1 to 4, there is illustrated an occlusion device according to the invention and indicated generally by the reference numeral 1. The occlusion device forms part of a large bore closure system of the invention, which is described in more detail below.

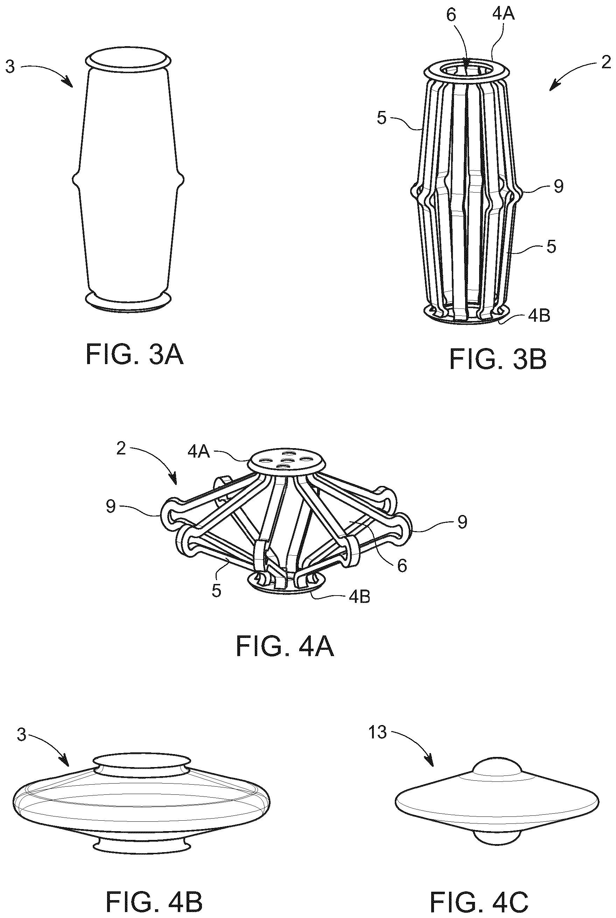

The occlusion device comprises a generally cylindrical frame 2 enclosed within a flexible fluid-impermeable sheath 3. In some embodiment, the device may include a balloon 13 which is dimensioned to fit within the frame and functions to receive and contain an injectable filler material (e.g., collagen) which may be injected into the balloon when the device has been deployed in-vivo and serves to maintain the shape of the deployed device and prevent fluid permeating through the deployed device. Figs 1 and 2 show the assembled occlusion device 1 with the frame disposed inside the sheath and the balloon disposed within the frame and Figures 3 and 4 show the frame, sheath and balloon prior to assembly. The frame 2 comprises a distal end 4A and proximal end 4B connected by eight elongated struts 5, defining a hollow lumen 6. In this embodiment, the frame 2 is formed from an extruded bioabsorbable co-polymer that is printed into the frame-shape. Each strut 5 has a distal end 7 with an inward inflection zone 8A, a proximal end 7 with an inward inflection zone 7A, and an outward inflection zone 9 disposed intermediate the distal end 7 and proximal end 8 that is configured to allow the strut fold outwardly during deployment, to form the waist section of the device. The configuration of the struts 5, in particular the inner inflection zones at each end of the strut and the outer inflection zone in the middle of the strut, urges the frame to compress into a "Chinese lantern" shape when axial torque is applied to either end of the frame thus compressing the frame from an elongated delivery configuration illustrated in Figure 1 to a compressed (squat) deployed configuration illustrated in Figure 2.

In this embodiment, the flexible sheath and frame are formed from a bioresorbable material (Such as Zeus Absorb - see slide attachment). The sheath is shaped to encase and closely adhere to the frame in a delivery configuration, as shown in Figure 3A, in which the sheath is shown in a relaxed non-tensioned configuration. As the sheath is formed from a flexible material, it can stretch to follow the shape of the frame as it is deployed from the shape shown in Figure 3B to the deployed shape of Figure 4A, where the sheath will be slightly tensioned but not enough to alter the shape of the frame.

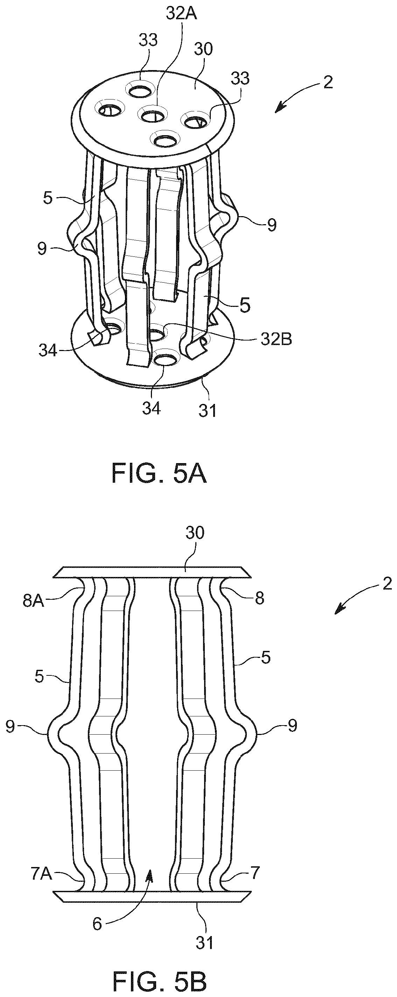

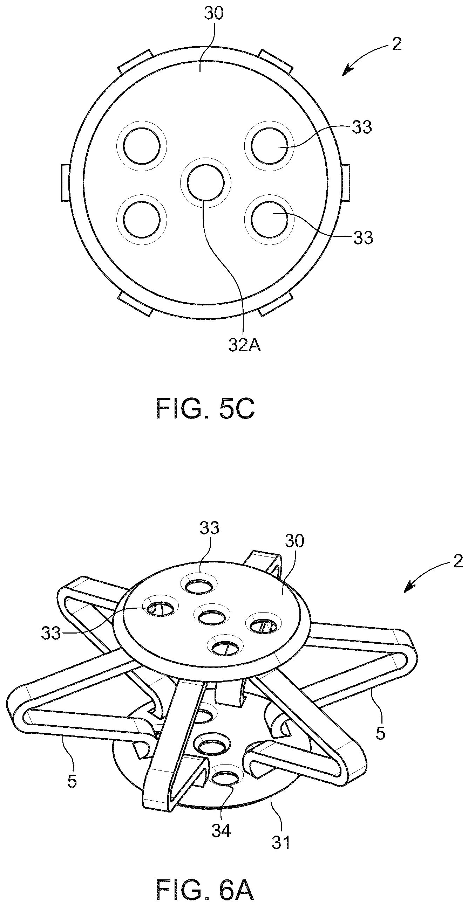

Referring to Figures 5 and 6, a specific embodiment of the occlusion device is illustrated in which parts identified with reference to the previous embodiments are assigned the same reference numerals. In this embodiment, the proximal end 4A and distal end 4B are provided by a proximal plate 30 and a distal plate 31, respectively. The proximal plate 30 has five apertures, including a central aperture 32A for receipt of a guidewire and four proximal apertures 33 (two on each side of the plate) for receipt of two control wires (not shown). The distal plate 31 has five apertures, including a central aperture 32B for receipt of a guidewire and four proximal apertures 34 (two on each side of the plate) for receipt of two control wires (not shown). In Figures 5A to 5C, the occlusion device is shown in the elongated delivery configuration, and in Figures 6A to 6C, the occlusion device is shown in the deployed radially expanded configuration.

Deployment of the occlusion device may employ one or more control wires that are operably coupled between a handle of the introducer device and the occlusion device. Generally, at least two control wires are employed, one connected to each side of the occlusion device to balance the torque being applied to the device and ensure that it deploys evenly. Each control wire may be a closed loop or a wire with free ends. In the embodiments described, the control wire is provided by a bioresorbable suture, but it will be appreciated that it may be provided by any other suitable elongated element.

Referring to the embodiment illustrated in Figures 7 and 8, part of a system of the invention is illustrated comprising an occlusion device 1 mounted on a guidewire 50 and two control wires 40. The introducer device and actuator for the control wires are omitted for clarity. In this embodiment, each control wire 40 is a closed loop part of which is spooled around the actuator rotor (not shown) and may be pulled in a first direction to deploy and brake/lock the device and pulled in an opposite direction to release the device and allow it to return to its un-tensioned elongated configuration. It will be appreciated also that the control wire does not have to be in the form of a closed loop and may be a wire with free ends each of which is attached to the rotor.

The system comprises two control wires 40 having a first end (not shown) attached to an actuator of the introducer device (e.g., a rotor) and a second end 42 coupled to the occlusion device. The second end of each control wire 40 has a first leg 47 that extends into the occlusion device through a proximal aperture 33 in the proximal plate 30, through the lumen 6 of the device, loops around the distal plate 31 via distal apertures 34, and a second leg 48 extends up through the lumen of the device and out through a second proximal aperture 34. An anchor element (bead 43) is disposed on the second leg 48 of the control wire just proximal of the proximal plate 30 and four braking elements (beads 44) are provided on the first leg 47 of the control wire, three in between the distal and proximal plates and one disposed on the control wire just proximal of the proximal plate. The anchor and braking beads disposed proximally of the proximal plate serve to maintain the position of the control wire relative to the occlusion device during delivery and prior to deployment.

Deployment of the device generally involves a user rotating a rotor on the handle of the introducer device which pulls the first leg 47 of the control wire in the direction of the arrow A in Figure 7A, resulting in the braking beads 44 being forced through the aperture in the proximal plate and the distal plate being pulled upwardly towards the proximal plate to deploy the device as shown in Figures 8A and 8B. In this configuration, the waisted section of the sidewall has extended radially outwardly to embed in the wall of the tissue tract anchoring the device in the tissue tract. The most distal braking bead 44A is located just proximal of the proximal plate and serves to maintain the device in the tensioned deployed configuration.

In the embodiment shown in Figure 8B, the device has been fully deployed (e.g., all four of the braking beads 44 have been pulled through the proximal aperture 33) but it will be appreciated that the level of deployment can be varied according to how far the control wire is pulled through the aperture. For example, if only the first braking bead 44 is pulled through the aperture 33, this would correlate with one third deployment. Likewise, if the first and second braking bead 44 are pulled through the aperture 33, this would correlate with two thirds deployment. In this way, a user can determine by tactile feedback how much the device has been deployed. Alternatively, or in addition, a graduated scale on the actuator rotor may also serve to indicate the degree of deployment.

The system of this embodiment may also be returned to the elongated delivery configuration as illustrated in Figures 9. This may be performed if the initial deployment is not successful, for example if it does not embed properly in the tissue tract or does not fully occlude the tissue tract, allowing the occlusion device to be re-sheathed or moved axially to a different position in the tissue tract. Referring to Figure 8B, deployment can be reversed by actuating the rotor on the introducer device to pull the second leg 48 of the control wire proximally in the direction of the arrow marked B, which causes the first leg 47 of the control wires and braking beads 44 to be pulled through the aperture in the proximal plate in the direction of the arrow marked C, which allows the distal end of the device to descend to return the device to the elongated delivery configuration shown in Figure 7. Once in this position, the device may be re-sheathed or moved axially and then re-deployed.

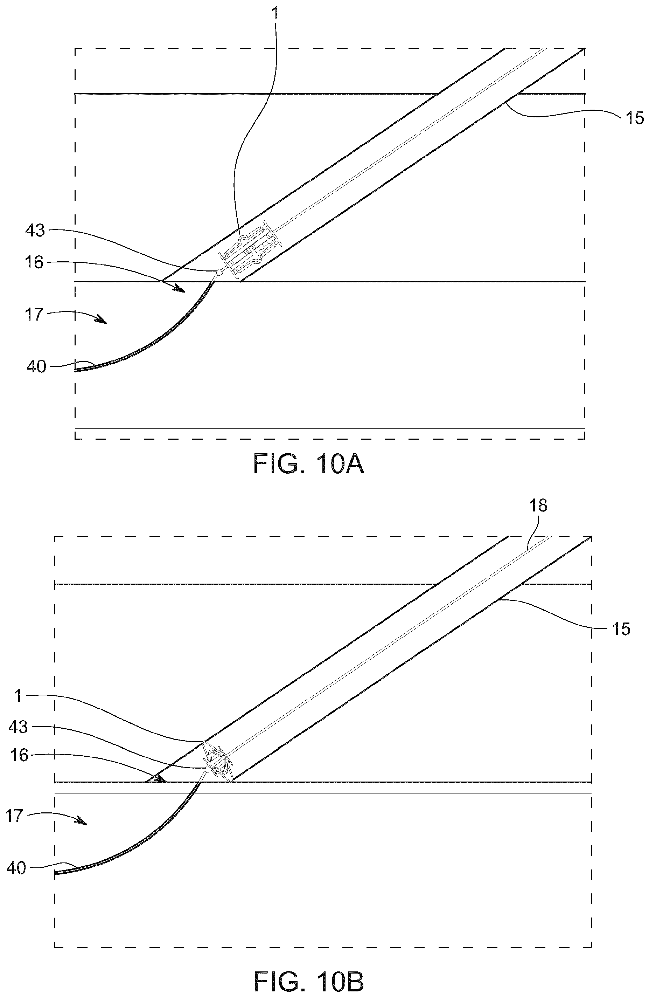

Figures 10A and 10B illustrate deployment of an occlusion device 1 according to the invention in a tissue tract 15 proximal of an opening 16 in a blood vessel 17. In Figure 10A, the occlusion device is shown released from an introducer (not shown) adjacent the opening of the blood vessel. A control wire 40 having an anchor bead 43 abutting a distal end 4A of the occlusion device is provided, with the proximal end of the wire (not shown) being attached to a deployment mechanism of the introducer device. Actuation of the deployment mechanism pulls the wire proximally, deploying the device from the elongated delivery configuration shown in Figure 10A to the compressed deployed configuration shown in Figure 10B, where the device anchors in a sidewall of the tissue tract to occlude the tissue tract. In practice, the introducer will remain in the tissue tract with a distal end of the introducer abutting a proximal end of the occlusion device while the wire is being pulled proximally.

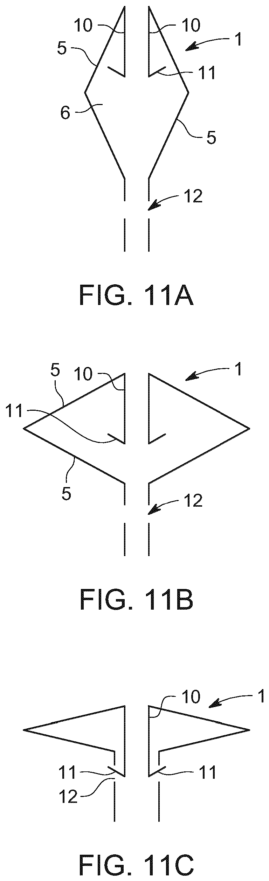

Figures 11A to 11B illustrate a locking mechanism for the occlusion device 1 comprising a locking arm 10 attached to a distal end of the frame 2 and having a hook 11 disposed on a free end of the locking arm 10. In a delivery configuration, the locking arm 10 is positioned within the hollow lumen 6 of the frame 2, and when the frame is adjusted into the deployed configuration shown in Figure 11C, the hook 11 engages an aperture 12 in a proximal end of the frame, to lock the frame in compressed deployed configuration. In other embodiments, the frame may be made from a shape memory material (such as nitinol) and may be biased into a deployed configuration, whereby the occlusion device may be constrained in an elongated delivery configuration within an introducer (described below) and will self-deploy when released from the introducer to anchor into the wall of a tissue tract.

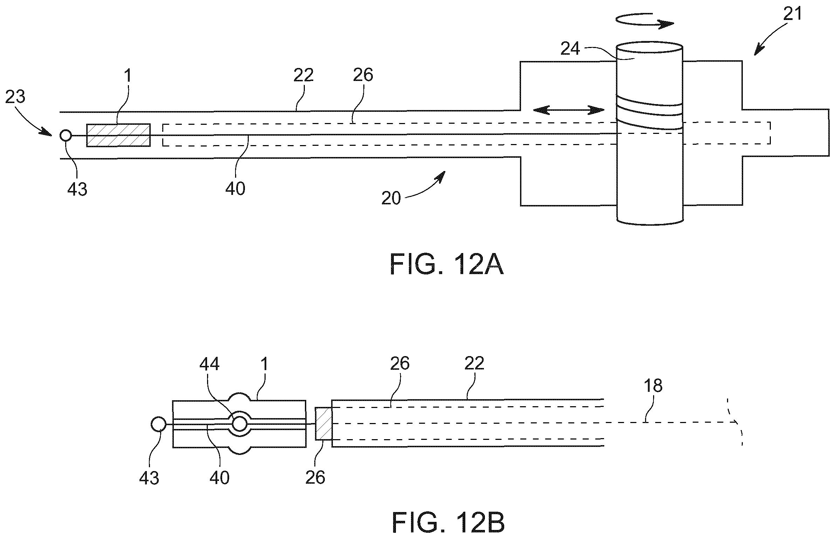

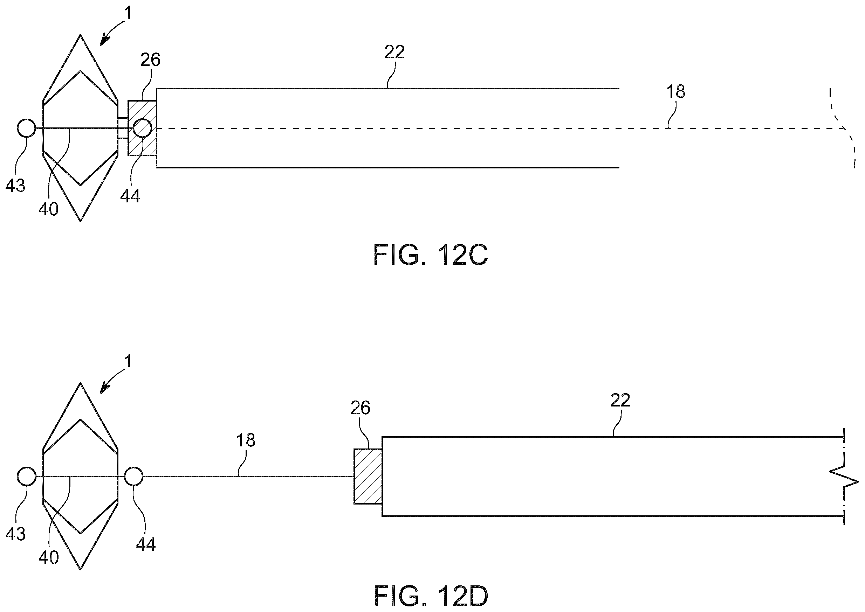

Figures 12A to 12D illustrates an introducer device forming part of a system according to the invention and indicated generally by the reference numeral 20, and its use to deliver and deploy an occlusion device. The introducer comprises a proximal handle 21 and a distal conduit 22 and a through lumen 23. The handle 21 comprises a rotor 24 which is rotatably mounted to the handle. An occlusion device 1 according to the disclosure is shown inserted into a distal end of the conduit 22 in an elongated delivery configuration. A control wire 40 is threaded through the occlusion device and has a distal end comprising an anchor bead 43 disposed just distal of the distal plate 31 and a braking bead 44 disposed on the wire between the end of the occlusion device, and a proximal end connected to the rotor 24 such that rotation of the rotor causes the control wire to be pulled proximally and the occlusion device to be gradually deployed, compressing the device and gradually increasing a maximum diameter of the device as it deploys. During deployment, the braking bead is pulled through an aperture in the proximal end of the occlusion device, where it acts as a brake to prevent the device reverting to its un-tensioned elongated configuration. Although not illustrated, the rotor/dial 24 may include a graduated scale of French bore size disposed circumferentially on a surface of the rotor. An example would an indication of 8F (2.7 mm) at 0 degrees (elongated delivery configuration), 12F (4.0 mm) at 90°, 16F (5.3 mm) at 180°, 20F (6.7 mm) at 270° and 24F (8.0 mm) at 360° (full rotation). The introducer device 20 also include an ejection element 26 that is axially mounted within the through lumen from a retracted configuration shown in Figure 12A to an ejection configuration shown in Figures 12B to 12D. Figures 12B to 12D illustrate the use of the introducer to deliver and deploy the device in which the needle tract is omitted for clarity. In Figure 12B the ejection element has been actuated to push the occlusion device 1 proud of a distal end of the distal conduit 23 into the tissue tract. The rotor (not shown) is then actuated to pull the control wire 40 proximally, resulting in the anchor element 43 pulling the distal end of the occlusion device proximally, while the proximal end of the occlusion device is maintained in situ due to it bearing against the distal end of the conduit 23. This results in the occlusion device being deployed as shown in Figure 12C. Once deployed and anchored into the wall of the tissue tract, the introducer device 20 is then withdrawn and the deployment wire is detached from the rotor and left in-situ attached to the occlusion device. As the occlusion device and deployment wire are made from bioresorbable materials, they will disintegrate over time at which point the opening in the blood vessel will have closed.

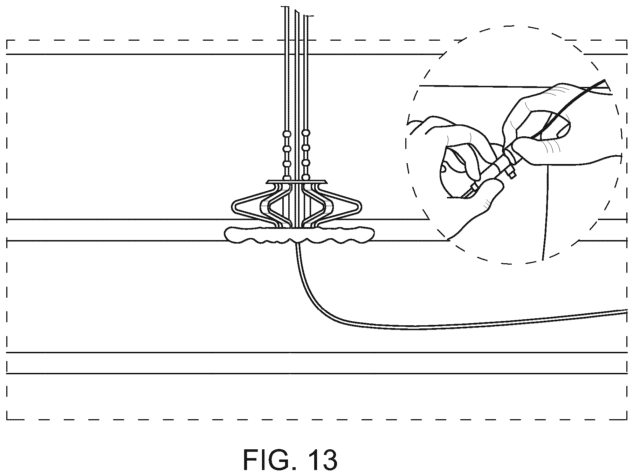

Figure. 13 is a schematic side elevational view of an occlusion device is a deployed configuration just proximal of an adventitial layer of the femoral artery.

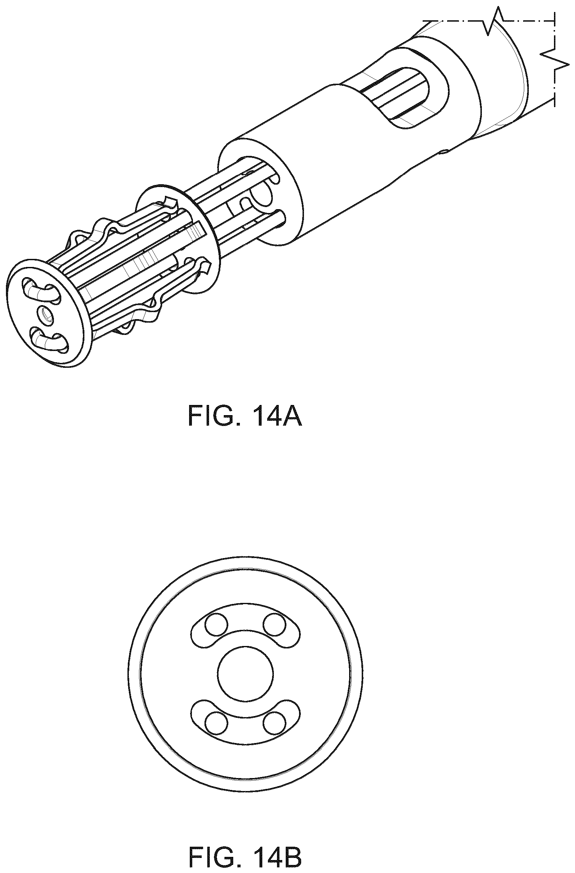

Figure 14A is a perspective view of a system of the invention including an occlusion device in an elongated delivery configuration, an introducer device, and two control wires (sutures) coupling the occlusion device and the actuator (not shown) of the occlusion device. The distal conduit part of the introducer device has a central through lumen (for receipt of the guidewire and the injectable filler material delivery conduit) and two crescent-shaped control wire lumens, each of which receives an ascending and descending parts of a control wires. The introducer device also comprises a reveal (window) on each side, each reveal providing a user access to the control wires to allow them to be cut when the occlusion device is deployed and anchored.

Figure 14B is an end elevational view of the distal conduit part of the introducer device of Figure 14A.

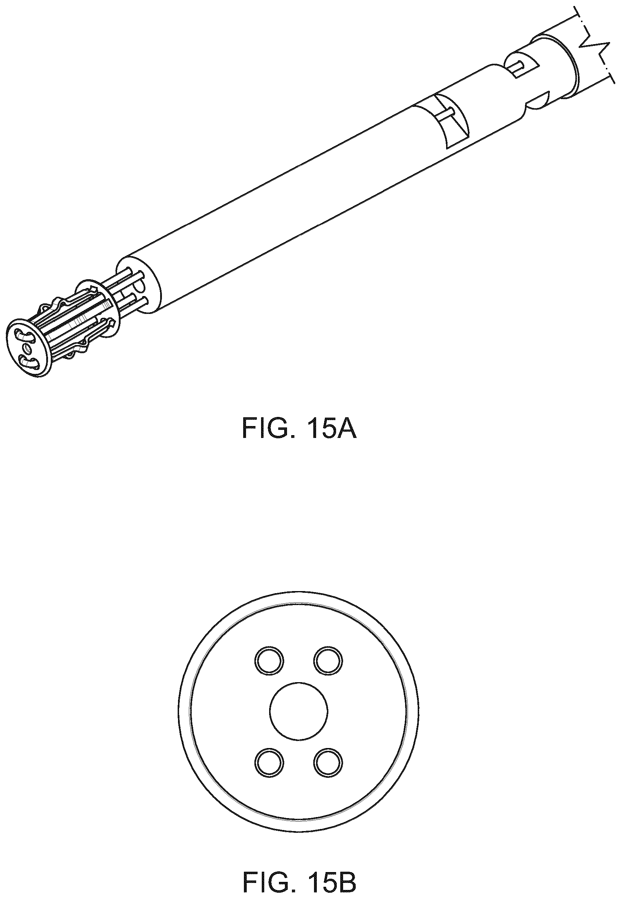

Figure 15A is a perspective view of an alternative embodiment of a system of the invention including an occlusion device in an elongated delivery configuration, an introducer device, and two control wires (sutures) coupling the occlusion device and the actuator (not shown) of the occlusion device. The distal conduit part of the introducer device has a central through lumen (for receipt of the guidewire and the injectable filler material delivery conduit) and four control wire lumens, each of which receives an ascending or descending parts of each control wire.

Figure 15B is an end elevational view of the distal conduit part of the introducer device of Figure 15A.

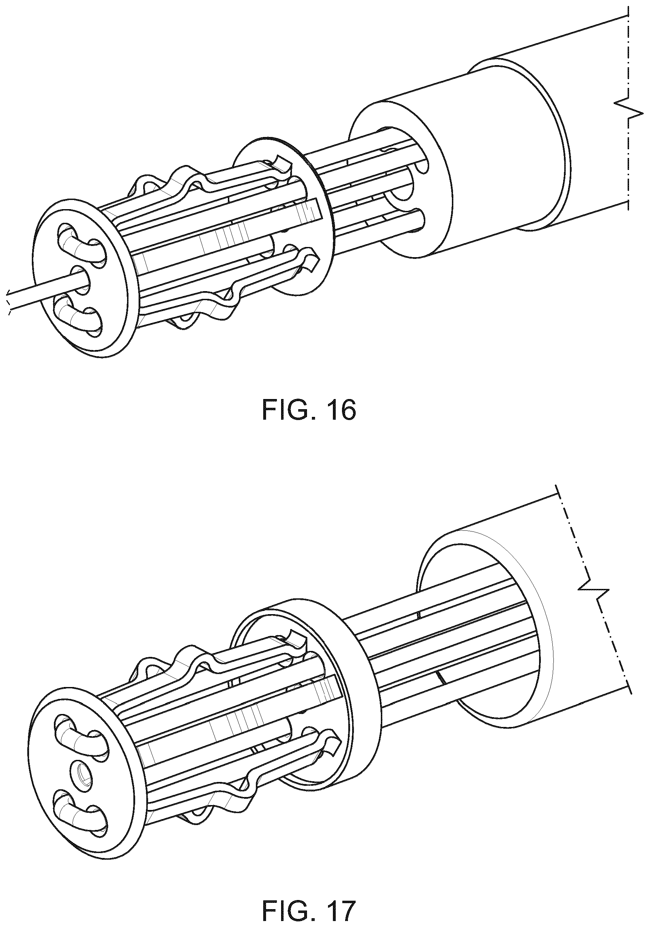

Figure 16 is a perspective view of an alternative embodiment of a system of the invention including an occlusion device in an elongated delivery configuration, an introducer device, and two control wires (sutures) coupling the occlusion device and the actuator (not shown) of the occlusion device. This embodiment is very similar to the embodiment of Figure 14 with the exception that the introducer device does not have windows to access the control wire lumen. Instead, the introducer device comprises a control wire release mechanism configured upon actuation to uncouple the control wires from the introducer device (e.g., from the actuator of the introducer device) to allow the occlusion device and introducer device to be uncoupled and the introducer device to be retracted and removed.

Figure 17 is a perspective view of an alternative embodiment of a system of the invention including an occlusion device in an elongated delivery configuration, an introducer device, and two control wires (sutures) coupling the occlusion device and the actuator (not shown) of the occlusion device. In this embodiment, the distal conduit of the introducer device has a single lumen configured to receive the guidewire, filler material conduit, and the control wires. The distal conduit typically comprises a cylindrical body, and end cap, and a coupling element to couple the cylindrical body and end cap configured to allow axial movement of the cylindrical body relative to the end cap. The coupling element may be a central strut. The end cap is generally dimensioned to receive a distal end of the occlusion body in a nesting relationship. The end cap may comprise a central aperture (or a plurality of apertures) to receive the control wires, guidewire and injectable filler material delivery conduit. The introducer device typically comprises an actuator to axially adjust the position of the cylindrical body relative to the end cap to expose the control wires to allow them to be cut.

In use, the following steps may be employed:
1- femoral artery is punctured using a small needle US Guided - (micro puncture or Kimmel needle)
2- soft J tip wire is introduced through the needle then a 6F (2.0 mm) sheath introduced over the wire
3- a wire is then reintroduced into the artery and a 9 or 10 F (3.0 mm or 3.3 mm) sheath then the large bore sheath is used to deliver the valve or do the case. Anything between 14 to 24 F (4.7 mm to 8.0 mm) sheath can be used depending on procedure.
4- Most cases are 14 to 18 F (4.7 mm to 6.0 mm) sheath - Large sheath is removed, and +/contralateral balloon is inflated proximal to sheath entrance hole (Iliac vessel)
5- SpioRAD Closure (occlusion) device is inserted over guidewire and expanded / rotated clockwise delivery @ 12F (4.0 mm) and Full deployment 24F-26F (8 mm - 8.7 mm) Nominal value
6- Syringe for filler material is attached to distal end of the introducer device and a syringe conduit is advanced through the lumen of the introducer device into the deployed closure device and filler material is injected into the closure device where the material serves to maintain the patency of the deployed device and also partially seeps through the walls of the closure device to adhere to the walls of the tissue tract anchoring the closure device in position. +/- Balloon is deflated. Ultrasound / Fluro Check device positioning and adherence
7- When haemostasis is achieved, then wire can be removed, and external dressing applied.

## Claims

1. A large bore extra-vascular closure system to occlude a large bore tissue tract proximal to a blood vessel, comprising:
an introducer device (20) having a through lumen (23) and a handle (31) comprising an actuator (24);
a bioresorbable occlusion device (1) having a distal end (4A, 31), a proximal end (4B, 30), a sidewall section comprising a waist section configured for outward inflection upon axial compression of the device, and a through lumen (6), wherein the occlusion device (1) is configured for adjustment from an elongated delivery configuration in which the occlusion device (1) is dimensioned to fit within the through lumen (23) of the introducer device (20) and a squat deployed configuration in which the waist section of the occlusion device (1) is expanded radially outwardly;
a bioresorbable control wire (40) operably connecting the actuator (24) of the introducer device and the distal end (4A, 31) of the occlusion device (1), wherein the actuator (24) is configured upon actuation to pull the control wire (40) proximally to deploy the occlusion device (1); and **characterized in that** the system further comprises
the distal end (31) of the occlusion device comprising two distal apertures (34) and the proximal end (30) of the device comprising two proximal apertures (33), in which the control wire (40) extends through a first of the proximal apertures (33), loops through the distal apertures (34) in the distal end (31) of the device and extends through a second of the proximal apertures (33);
an injectable filler material configured for injection into the hollow lumen (6) of the occlusion device (1) when deployed, wherein the injectable filler material is bioresorbable.

2. The large bore extra-vascular closure system according to Claim 1, in which the injectable filler material is thermoresponsive.

3. The large bore extra-vascular closure system according to Claim 1 or 2, including a syringe device comprising a delivery conduit configured to extend through the through lumen (23) of the introducer device (20) to deliver injectable filler material into the through lumen (6) of the bioresorbable occlusion device (1) when the bioresorbable occlusion device (1) is deployed.

4. The large bore extra-vascular closure system according to Claim 3, in which the delivery conduit of the syringe device comprises a non-stick coating configured to inhibit the injectable filler material sticking to an inside of the delivery conduit.

5. The large bore extra-vascular closure system according to any preceding Claim, in which the sidewall section of the bioresorbable occlusion device (1) comprises a plurality of struts (5) providing a sidewall section with an open framework.

6. The large bore extra-vascular closure system according to any preceding Claim, in which the bioresorbable control wire (40) extends through a proximal aperture (33) in the proximal end (30) of the occlusion device (1) and through a distal aperture (34) in the distal end (31) of the occlusion device and comprises an anchor element (43) that cannot pass through the distal or proximal aperture (34,33), and braking element (44) dimensioned for a friction fit through the distal or proximal aperture (34,33).

7. The large bore extra-vascular closure system according to any preceding Claim, including a plurality of control wires (40) connecting the actuator (24) of the introducer device and the distal end (4A, 31) of the occlusion device (1).

8. The large bore extra-vascular closure system according to any preceding Claim, in which the control wire (40) is a closed loop.

9. The large bore extra-vascular closure system according to any preceding Claim, in which the actuator (24) is a rotor and the control wire (40) is mounted to the rotor (24) whereby rotation of the rotor (24) effects movement of the control wire (40) relative to the introducer device (20).

10. The large bore extra-vascular closure system according to Claim 9, in which the rotor (24) comprises a graduated scale disposed on a circumference of the rotor (24) indicating a range of large bore sizes.

11. The large bore extra-vascular closure system according to any of Claims 5 to 10, in which the waisted section of the sidewall is formed by an outward inflection zone (9) disposed intermediate the ends (7, 8) of each elongated strut (5) configured to allow the strut (5) fold radially outwardly during deployment.

12. The large bore extra-vascular closure system according to Claim 11, in which each of the elongated struts (5) comprises an inward inflection zone (7A,8A) disposed at each end (7, 8) of the strut configured to allow an adjacent section of the strut (5) bend radially outwardly during deployment.

13. The large bore extra-vascular closure system according to any preceding Claim, in which the introducer device (20) comprises a distal conduit (22) housing the through lumen (23) and an outer retractable sheath configured for axial adjustment relative to the distal conduit (22) from a delivery configuration in which a distal end of the outer retractable sheath extends distally of the distal end of the distal conduit (22) providing a space for receipt of the occlusion device (1) and a deployment configuration where the outer retractable sheath is retracted distally to expose the occlusion device (1) to the surrounding wall of the tissue tract.

14. The large bore extra-vascular closure system according to any preceding Claim, in which the introducer device (20) comprises a distal conduit housing the through lumen (23) and one or more control wire lumens to house the or each control wires (40).

15. The large bore extra-vascular closure system according to any preceding Claim, in which the distal end of the occlusion device (1) has a peripheral flange section that extends radially outwardly of a distal end of the sidewall section, wherein the distal end or peripheral flange section comprises a deformable material.

## Patentansprüche

1. Extravasales Verschlusssystem mit großem Durchmesser zum Verschließen eines Gewebetrakts mit großem Durchmesser in der Nähe eines Blutgefäßes, das Folgendes umfasst:
eine Einführvorrichtung (20) mit einem Durchgangslumen (23) und einem Griff (31) mit einem Stellelement (24),
eine bioresorbierbare Verschlussvorrichtung (1) mit einem distalen Ende (4A, 31), einem proximalen Ende (4B, 30), einem Seitenwandabschnitt, der einen Gelenkabschnitt umfasst, welcher so konfiguriert ist, dass er sich bei axialer Kompression der Vorrichtung nach außen krümmt, und einem Durchgangslumen (6), wobei die Verschlussvorrichtung (1) zum Verstellen aus einer gestreckten Abgabekonfiguration, in der die Verschlussvorrichtung (1) so bemessen ist, dass sie in das Durchgangslumen (23) der Einführvorrichtung (20) passt, und einer gedrungenen Einsatzkonfiguration konfiguriert ist, in der der Gelenkabschnitt der Verschlussvorrichtung (1) radial nach außen ausgeklappt ist,
einen bioresorbierbaren Steuerdraht (40), der das Stellelement (24) der Einführvorrichtung und das distale Ende (4A, 31) der Verschlussvorrichtung (1) wirkverbindet,
wobei das Stellelement (24) so konfiguriert ist, dass es bei Betätigung proximal am Steuerdraht (40) zieht und so die Verschlussvorrichtung (1) einsetzt, und
**dadurch gekennzeichnet, dass** das System ferner Folgendes umfasst:
das distale Ende (31) der Verschlussvorrichtung zwei distale Öffnungen (34) und das proximale Ende (30) der Vorrichtung zwei proximale Öffnungen (33) umfasst, wobei der Steuerdraht (40) durch eine erste der proximalen Öffnungen (33) verläuft, über die distalen Öffnungen (34) im distalen Ende (31) der Vorrichtung eine Schlaufe bildet und durch eine zweite der proximalen Öffnungen (33) verläuft,
ein injizierbares Füllmaterial, das so konfiguriert ist, dass es in das Hohllumen (6) der eingesetzten Verschlussvorrichtung (1) injiziert wird, wobei das injizierbare Füllmaterial bioresorbierbar ist.

2. Extravasales Verschlusssystem mit großem Durchmesser nach Anspruch 1, bei dem das injizierbare Füllmaterial thermoresponsiv ist.

3. Extravasales Verschlusssystem mit großem Durchmesser nach Anspruch 1 oder 2 mit einer Spritzenvorrichtung, die einen Abgabekanal umfasst, der so konfiguriert ist, dass er durch das Durchgangslumen (23) der Einführvorrichtung (20) verläuft und so injizierbares Füllmaterial in das Durchgangslumen (6) der bioresorbierbaren Verschlussvorrichtung (1) abgibt, wenn die bioresorbierbare Verschlussvorrichtung (1) eingesetzt wird.

4. Extravasales Verschlusssystem mit großem Durchmesser nach Anspruch 3, bei dem der Abgabekanal der Spritzenvorrichtung eine Antihaftbeschichtung umfasst, die so konfiguriert ist, dass sie verhindert, dass das injizierbare Füllmaterial innen am Abgabekanal haften bleibt.

5. Extravasales Verschlusssystem mit großem Durchmesser nach einem der vorhergehenden Ansprüche, bei dem der Seitenwandabschnitt der bioresorbierbaren Verschlussvorrichtung (1) mehrere Streben (5) umfasst, die einen Seitenwandabschnitt mit einem offenen Gerüst bereitstellen.

6. Extravasales Verschlusssystem mit großem Durchmesser nach einem der vorhergehenden Ansprüche, bei dem der bioresorbierbare Steuerdraht (40) durch eine proximale Öffnung (33) im proximalen Ende (30) der Verschlussvorrichtung (1) und durch eine distale Öffnung (34) im distalen Ende (31) der Verschlussvorrichtung verläuft und ein Ankerelement (43), das die distale oder die proximale Öffnung (34, 33) nicht passieren kann, und ein Bremselement (44) umfasst, das für eine Friktionspassung in der distalen oder der proximalen Öffnung (34, 33) bemessen ist.

7. Extravasales Verschlusssystem mit großem Durchmesser nach einem der vorhergehenden Ansprüche mit mehreren Steuerdrähten (40), die das Stellelement (24) der Einführvorrichtung und das distale Ende (4A, 31) der Verschlussvorrichtung (1) verbinden.

8. Extravasales Verschlusssystem mit großem Durchmesser nach einem der vorhergehenden Ansprüche, bei dem es sich bei dem Steuerdraht um eine geschlossene Schlaufe handelt.

9. Extravasales Verschlusssystem mit großem Durchmesser nach einem der vorhergehenden Ansprüche, bei dem es sich bei dem Stellelement (24) um einen Rotor handelt und der Steuerdraht (40) an dem Rotor (24) angebracht ist, so dass eine Rotation des Rotors (24) eine Bewegung des Steuerdrahts (40) in Bezug auf die Einführvorrichtung (20) bewirkt.

10. Extravasales Verschlusssystem mit großem Durchmesser nach Anspruch 9, bei dem der Rotor (24) eine Teilstrichskala umfasst, die an einem Umfang des Rotors (24) angeordnet ist und einen Größenbereich von großen Durchmessern angibt.

11. Extravasales Verschlusssystem mit großem Durchmesser nach einem der Ansprüche 5 bis 10, bei dem der Gelenkabschnitt der Seitenwand von einer sich nach außen krümmenden Zone (9) gebildet wird, die zwischen den Enden (7, 8) jeder länglichen Strebe (5) angeordnet und so konfiguriert ist, dass die Strebe (5) beim Einsatz radial nach außen hin zusammenklappen kann.

12. Extravasales Verschlusssystem mit großem Durchmesser nach Anspruch 11, bei dem jede der länglichen Streben (5) eine sich nach innen krümmende Zone (7A, 8A) umfasst, die an jedem Ende (7, 8) der Strebe angeordnet und so konfiguriert ist, dass sich ein benachbarter Abschnitt der Strebe (5) beim Einsatz radial nach außen hin biegen kann.

13. Extravasales Verschlusssystem mit großem Durchmesser nach einem der vorhergehenden Ansprüche, bei dem die Einführvorrichtung (20) einen distalen Kanal (22), in dem das Durchgangslumen (23) untergebracht ist, und eine zurückziehbare Außenhülse umfasst, die so konfiguriert ist, dass sie sich in Bezug auf den distalen Kanal (22) axial aus einer Abgabekonfiguration, in der ein distales Ende der zurückziehbaren Außenhülse distal zum distalen Ende des distalen Kanals (22) verläuft und Platz zum Aufnehmen der Verschlussvorrichtung (1) bietet, und einer Einsatzkonfiguration verstellen lässt, bei der die zurückziehbare Außenhülse distal zurückgezogen ist und so die Verschlussvorrichtung (1) der umgebenden Wand des Gewebetrakts gegenüber freiliegt.

14. Extravasales Verschlusssystem mit großem Durchmesser nach einem der vorhergehenden Ansprüche, bei dem die Einführvorrichtung (20) einen distalen Kanal umfasst, in dem das Durchgangslumen (23) und ein oder mehrere Steuerdrahtlumen zum Unterbringen des oder jedes Steuerdrahtes (40) untergebracht sind.

15. Extravasales Verschlusssystem mit großem Durchmesser nach einem der vorhergehenden Ansprüche, bei dem das distale Ende der Verschlussvorrichtung (1) einen peripheren Flanschabschnitt aufweist, der an einem distalen Ende des Seitenwandabschnitts radial nach außen verläuft, wobei das distale Ende oder der periphere Flanschabschnitt ein verformbares Material umfasst.

## Revendications

1. Système de fermeture extravasculaire de grand alésage pour occlure un tractus tissulaire de grand alésage proximal par rapport à un vaisseau sanguin, comprenant :
un dispositif introducteur (20) ayant une lumière traversante (23) et une poignée (31) comprenant un actionneur (24) ;
un dispositif d'occlusion biorésorbable (1) ayant une extrémité distale (4A, 31), une extrémité proximale (4B, 30), une section de paroi latérale comprenant une section de cambrure configurée pour une inflexion vers l'extérieur lors de la compression axiale du dispositif, et une lumière traversante (6), dans lequel le dispositif d'occlusion (1) est configuré pour un ajustement à partir d'une configuration de délivrance allongée dans laquelle le dispositif d'occlusion (1) est dimensionné pour s'adapter au sein de la lumière traversante (23) du dispositif introducteur (20) et d'une configuration déployée fléchie dans laquelle la section de cambrure du dispositif d'occlusion (1) est déployée de manière radiale vers l'extérieur ;
un fil de commande biorésorbable (40) connectant de manière fonctionnelle l'actionneur (24) du dispositif introducteur et l'extrémité distale (4A, 31) du dispositif d'occlusion (1), dans lequel l'actionneur (24) est configuré lors de l'actionnement pour tirer le fil de commande (40) de manière proximale pour déployer le dispositif d'occlusion (1) ; et **caractérisé en ce que** le système comprend en outre
l'extrémité distale (31) du dispositif d'occlusion comprenant deux ouvertures distales (34) et l'extrémité proximale (30) du dispositif comprenant deux ouvertures proximales (33), dans lesquelles le fil de commande (40) s'étend à travers une première des ouvertures proximales (33), passe en boucle à travers les ouvertures distales (34) dans l'extrémité distale (31) du dispositif et s'étend à travers une deuxième des ouvertures proximales (33) ;
un matériau de remplissage injectable configuré pour être injecté dans la lumière creuse (6) du dispositif d'occlusion (1) lorsqu'il est déployé, dans lequel le matériau de remplissage injectable est biorésorbable.

2. Système de fermeture extravasculaire à grand alésage selon la revendication 1, dans lequel le matériau de remplissage injectable est thermosensible.

3. Système de fermeture extravasculaire de grand alésage selon la revendication 1 ou 2, incluant un dispositif de seringue comprenant un conduit de délivrance configuré pour s'étendre à travers la lumière traversante (23) du dispositif introducteur (20) pour distribuer un matériau de remplissage injectable dans la lumière traversante (6) du dispositif d'occlusion biorésorbable (1) lorsque le dispositif d'occlusion biorésorbable (1) est déployé.

4. Système de fermeture extravasculaire de grand alésage selon la revendication 3, dans lequel le conduit de délivrance du dispositif de seringue comprend un revêtement antiadhésif configuré pour empêcher que le matériau de remplissage injectable colle à un intérieur du conduit de distribution.

5. Système de fermeture extravasculaire de grand alésage selon l'une quelconque des revendications précédentes, dans lequel la section de paroi latérale du dispositif d'occlusion biorésorbable (1) comprend une pluralité d'entretoises (5) fournissant une section de paroi latérale avec une ossature ouverte.

6. Système de fermeture extravasculaire de grand alésage selon l'une quelconque des revendications précédentes, dans lequel le fil de commande biorésorbable (40) s'étend à travers une ouverture proximale (33) dans l'extrémité proximale (30) du dispositif d'occlusion (1) et à travers une ouverture distale (34) dans l'extrémité distale (31) du dispositif d'occlusion et comprend un élément d'ancre (43) qui ne peut pas passer à travers l'ouverture distale ou proximale (34, 33), et un élément de freinage (44) dimensionné pour un ajustement par frottement à travers l'ouverture distale ou proximale (34, 33).

7. Système de fermeture extravasculaire de grand alésage selon l'une quelconque des revendications précédentes, incluant une pluralité de fils de commande (40) connectant l'actionneur (24) du dispositif introducteur et l'extrémité distale (4A, 31) du dispositif d'occlusion (1).

8. Système de fermeture extravasculaire de grand alésage selon l'une quelconque des revendications précédentes, dans lequel le fil de commande (40) est une boucle fermée.

9. Système de fermeture extravasculaire de grand alésage selon l'une quelconque des revendications précédentes, dans lequel l'actionneur (24) est un rotor et le fil de commande (40) est monté sur le rotor (24) par quoi la rotation du rotor (24) provoque un mouvement du fil de commande (40) par rapport au dispositif introducteur (20).

10. Système de fermeture extravasculaire de grand alésage selon la revendication 9, dans lequel le rotor (24) comprend une échelle graduée disposée sur une circonférence du rotor (24) indiquant une gamme de tailles de grand alésage.

11. Système de fermeture extravasculaire de grand alésage selon l'une quelconque des revendications 5 à 10, dans lequel la section cambrée de la paroi latérale est formée par une zone d'inflexion vers l'extérieur (9) disposée entre les extrémités (7, 8) de chaque entretoise allongée (5) configurée pour permettre à l'entretoise (5) de se plier de manière radiale vers l'extérieur pendant le déploiement.

12. Système de fermeture extravasculaire de grand alésage selon la revendication 11, dans lequel chacune des entretoises allongées (5) comprend une zone d'inflexion vers l'intérieur (7A, 8A) disposée au niveau de chaque extrémité (7, 8) de l'entretoise configurée pour permettre à une section adjacente de l'entretoise (5) de se plier de manière radiale vers l'extérieur pendant le déploiement.

13. Système de fermeture extravasculaire de grand alésage selon l'une quelconque des revendications précédentes, dans lequel le dispositif introducteur (20) comprend un conduit distal (22) logeant la lumière traversante (23) et une gaine rétractable externe configurée pour un ajustement axial par rapport au conduit distal (22) à partir d'une configuration de délivrance dans laquelle une extrémité distale de la gaine rétractable externe s'étend de manière distale par rapport à l'extrémité distale du conduit distal (22) fournissant un espace pour la réception du dispositif d'occlusion (1) et une configuration de déploiement où la gaine rétractable externe est rétractée de manière distale pour exposer le dispositif d'occlusion (1) à la paroi environnante du tractus tissulaire.

14. Système de fermeture extravasculaire de grand alésage selon l'une quelconque des revendications précédentes, dans lequel le dispositif introducteur (20) comprend un conduit distal logeant la lumière traversante (23) et une ou plusieurs lumières de fil de commande pour loger le ou chaque fil de commande (40).

15. Système de fermeture extravasculaire de grand alésage selon l'une quelconque des revendications précédentes, dans lequel l'extrémité distale du dispositif d'occlusion (1) a une section de bride périphérique qui s'étend vers l'extérieur de manière radiale d'une extrémité distale de la section de paroi latérale, dans lequel l'extrémité distale ou la section de bride périphérique comprend un matériau déformable.
